# EUROPÄISCHE PATENTSCHRIFT

(11) **EP 0 621 252 B1**
(45) Veröffentlichungstag und Bekanntmachung des Hinweises auf die Patenterteilung: **12.03.1997**
(21) Anmeldenummer: 94105136.9
(22) Anmeldetag: 31.03.1994
(51) Int. Cl.: C07C 37/20, C07C 39/16

(54) **Ionenaustauscherschüttungen von zwei Schichten für die Bisphenol-A-Synthese**
Twolayer ionexchanger for bisphenol-A synthesis
Echangeuses d'ions à deux couches pour la synthèse de bisphénol-A

(30) Priorität: 13.04.1993 DE 4312039
(43) Veröffentlichungstag der Anmeldung: 26.10.1994
(73) Patentinhaber: BAYER AG, 51368 Leverkusen (DE)
(72) Erfinder: Berg, Klaus, Dr., D-47798 Krefeld (DE); Malamet, Georg, Dr., D-47800 Krefeld (DE); Eitel, Alfred, Dr., D-41539 Dormagen (DE); Wulff, Claus, Dr., D-47800 Krefeld (DE)

(56) Entgegenhaltungen:
- US-A- 4 400 555
- DATABASE WPI, Week 9207, Derwent Publications Ltd., London, GB; AN 92-053920 & JP-A-4 001 149
- CHEMICAL ABSTRACTS, Bd. 118, Nr. 16, 19. April 1993, Columbus, Ohio, US; Zusammenfassung Nr. 148084f, M. KIEDIK et al: "Effect of crosslinking of cation exchangers on their catalytic activity in the condensation of phenol with acetone", Seite 1, Spalte 2
- CHEMICAL ABSTRACTS, Bd. 116, Nr. 7, 17. Februar 1992, Columbus, Ohio, US; Zusammenfassung Nr. 58942, Seite 818, Spalte 1; & PL-A-153 148
- CHEMICAL ABSTRACTS, Bd. 120, 1994, Columbus, Ohio, US; Zusammenfassung Nr. 247789n, Seite 138, Spalte 1; & JP-A-5 317 686

## Beschreibung

Die Erfindung betrifft ein Verfahren zur Erhöhung (Verbesserung) der Raum-Zeit-Ausbeute bei Festbettreaktoren bei der Bisphenol-A Herstellung.

Verfahren zur Synthese von Bisphenol-A mittels Ionenaustauscherkatalyse sind bekannt z.B. US-A 4 391 997, 4 400 555, 4 590 303, JP-A 8 272 972, PL-A 96 436, CS-A 183 069, EP-A 210 366 etc.

Es ist bekannt, daß bei der großtechnischen Herstellung von Bisphenol-A (BPA) ein Gemisch aus überschüssigem Phenol und Aceton durch einen zylindrischen Festbettreaktor, der mit sulfonsauren Ionenaustauscherharzen (auf Polystyrol-Basis) gefüllt ist, geleitet und anschließend der Aufarbeitung zugeführt wird. Wahlweise kann die Strömungsrichtung des Gemisches von oben nach unten oder von unten nach oben gewählt werden. Jede dieser Einspeiserichtungen hat Vor- und Nachteile.

Bei einer Einspeiserichtung von oben nach unten stellt der Druckverlust durch das Ionenaustauscherbett wegen der entstehenden Durchsatzbehinderung ein entscheidendes Problem dar. Der Druckverlust kann z.B. durch die Kompressibilität des verwendeten Ionenaustauscherharzes hervorgerufen werden. Die kugeligen Harzkörner können unter Druck linsenartig verformt werden und so zu einer exponentiellen Verminderung des Durchsatzes führen. Ein Festpressen der Katalysatorschüttung kann die Bildung von Strömungskanälen begünstigen, so daß der Reaktor nicht gleichmäßig durchströmt werden kann. Ein vollständiges Ausnutzen der gesamten eingesetzten Katalysatormenge ist so nicht gewährleistet.

Es wurde nun ein Verfahren gefunden, bei dem der Druckverlust bei einer großtechnischen Herstellung von Bisphenol-A aus Aceton und Phenol in einem zylindrischen Festbettreaktor, gefüllt mit sulfonsauren Ionenaustauscherharzen bis in den Bereich großer Einspeisemengen stark begrenzt werden kann. So kann eine erhebliche Kapazitätsausweitung des Festbettreaktors erreicht werden. Die Kapazität ist nicht mehr durch die Einspeisemenge und den entsprechenden Druckverlusten begrenzt. Sie wird durch den Acetonumsatz bestimmt.

Eine derartige hydraulische Problematik ist insbesondere bei sulfonsauren Ionenaustauschern mit niedrigem Vernetzungsgrad (≤2 %) zu registrieren. Andererseits stellen gerade diese Ionenaustauscher bei der BPA-Synthese das Optimum hinsichtlich Reaktivität und Selektivität dar.

Bei höhervernetzten Ionenaustauscherharzen (≥2,5 %) nehmen zwar die hydraulischen Probleme der entsprechenden Schüttungen mit zunehmendem Vernetzungsgrad ab, gleichzeitig verringert sich aber auch die Reaktivität und Selektivität derartiger Harztypen bei der BPA-Synthese in beträchtlichem Maße.

Eine Möglichkeit zur Verbesserung der hydraulischen Qualität niedrigvernetzter Harzschüttungen besteht in der teilweisen Belegung der Sulfonsäuregruppen mit Kationen. Besonders vorteilhaft ist die teilweise Belegung mit ⁺NH₃-CH₂-CH₂-SH oder ähnlichen Systemen, wie z.B. in der DE-A 3 619 450 und US-A 3 394 089 beschrieben. Neben einer Versprödung und damit größeren Starrheit des Ionenaustauschers wird zusätzlich ein katalytischer Effekt der Gruppen bei der BPA-Synthese beobachtet.

Allerdings ist die Standzeit derartiger Systeme durch die Desaktivierung der cokatalytischen Einheit gegenüber unmodifizierten Harzsystemen ca. um den Faktor 10 verkrüzt und damit unökonomisch. Die anschließend notwendige Regeneration der großen Mengen Lewatit ist zeitraubend, kostenintensiv und muß zur Aufrechterhaltung der BPA-Produktion durch eine gleich große Menge an frischem Ionenaustauscher ersetzt werden.

Es wurde nun ein Harzschüttungsmodell gefunden, das die Vorteile der jeweiligen Harze in optimaler Weise miteinander verknüpft: Das Ionenaustauscherbett im Reaktor besteht in der unteren Schicht (volumenmäßig 75-85 %) aus einem unmodifizierten, sulfonsauren Ionenaustauscher mit niedrigem Vernetzungsgrad (≤2 %). Die obere Schicht (volumenmäßig 15-25 %) kann bestehen aus a) einem höhervernetzten Ionenaustauschertyp (Vernetzungsgrad ≥2 %) oder b) aus einem niedrigvernetzten Harztyp (Vernetzungsgrad ≤2 %), dessen Sulfonsäuregruppen zu 1-25 Mol-% mit Alkyl-SH enthaltenden Spezies (z.B. Mercaptoethylaminen, wie in DE-A 3 619 450 oder US-PS 334 940 089 beschrieben) belegt worden sind (ionische Fixierung).

Gegenstand der Erfindung sind daher Ionenaustauscherschüttungen zur Erhöhung der Raum-Zeit-Ausbeute der Festbettreaktoren bei der Bisphenol A-Herstellung aus Phenol und Aceton in zylindrischen Festbettreaktoren, die mit sulfonsauren Ionenaustauscherharzen von gelförmigem oder makroporösem Typ gefüllt sind, dadurch gekennzeichnet, daß
a) die untere Schicht der Schüttung aus einem niedrigvernetzten Harztyp (Vernetzungsgrad (≤2 %) besteht und volumenmäßig 75-85 % der Gesamtschüttung ausmacht und
b) die obere Schicht der Schüttung mit einem Volumenanteil von 15-25 % entweder aus einem höhervernetzten Harztyp (Vernetzungsgrad ≥2 % ≤4 %), dessen Sulfonsäuregruppen zu 1-25 Mol-% mit Alkyl-SH-Einheiten enthaltenden Spezies (ionisch fixiert) belegt sein können, besteht oder
c) aus einem niedrigvernetzten Harz (Vernetzungsgrad ≤2 %), dessen Sulfonsäuregruppen zu 1-25 Mol-% mit Alkyl-SH-Einheiten enthaltenden Spezies (ionisch fixiert) belegt sind.

Erfindungsgemäße Harzschüttungen verhalten sich hydraulisch so, als ob die obere, starre Harzschicht die alleinige Füllung des Reaktors darstellt, d.h. die Kapazität des Reaktors wird im wesentlichen nicht mehr durch die Hydraulik der Füllung, sondern durch den Acetonumsatz bestimmt.

Der Schichttyp aus a) und c) zeigt bei der BPA-Synthese neben seinen guten hydraulischen Eigenschaften überraschenderweise auch noch das ausgezeichnete Reaktivitäts- und Selektivitätsverhalten einer Harzschüttung, die ausschließlich aus einem niedrigvernetzten Ionenaustauschertyp (≤2 %) besteht, an dessen Sulfonsäuregruppen partiell Alkyl-SH-Spezies ionisch fixiert sind. Bei einer Desaktivierung der obersten Ionenaustauscherschicht beim Typ aus a) und c) kann diese aufgrund ihres geringen Volumens in ökonomisch vertretbarer Weise regeneriert oder entsorgt werden.

Beim Schichttyp aus a) und b), der zwar exzellente hydraulische Eigenschaften, aber nicht das hervorragende Selektivitäts- und Reaktivitätsverhalten des Schichttyps aus a) und c) aufweist, kann durch partielles Belegen der Sulfonsäureeinheiten (1-25 Mol-%) der höhervernetzten, oberen Harzschicht mit Alkyl-SH-Einheiten (z.B. Mercaptoethylamin, Thiazolidin) auf ein fast ebenbürtiges Niveau gebracht werden.

Die Präparation der modifizierten als auch unmodifizierten Ionenaustauscherharze erfolgt auf bekannten Wegen (wie z.B. in der US-PS 3 394 089 oder in der DE-A 3 619 450 beschrieben). Die Herstellung des Bisphenol-A und dessen Aufarbeitung erfolgt kontinuierlich, wobei die Verfahrensweise und die erforderlichen Apparate bekannt sind.

Die erfindungsgemäßen Ionenaustauscherschüttungen für die Bisphenol-A Synthese stellen ein Optimum an Hydraulik, Selektivität und Reaktivität bei der BPA-Synthese.

### Beispiele

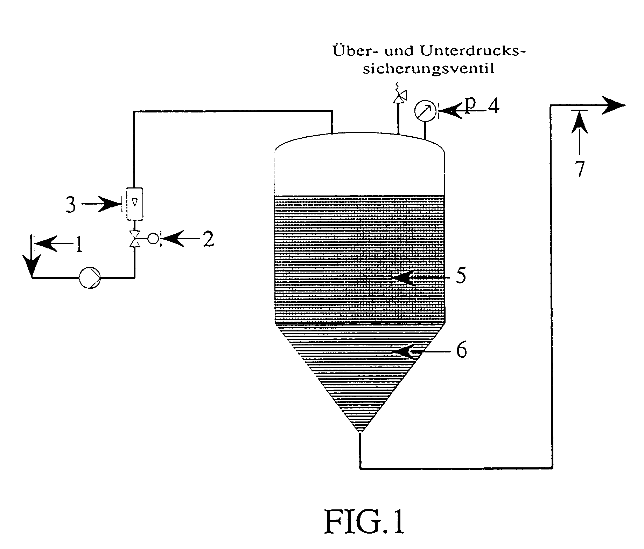

### Abbildung 1 (Fig. 1) verdeutlicht den Reaktortyp, Reaktoraufbau und -betriebsweise.

Über eine Leitung (1) wird ein Gemisch aus Phenol, recyclisierter Mutterlauge (bestehend aus Phenol, Bisphenol-A und Nebenprodukten) und Aceton von oben auf den Reaktor gegeben. Der Reaktor ist üblicherweise zu 50 bis 80 % seines Gesamtvolumens mit Ionenaustauscher (5) gefüllt. Im unteren konischen Teil (6) des Reaktors befindet sich eine Schicht aus mineralischem Material als Träger für das Harzbett. Das Reaktionsgemisch strömt von oben nach unten durch das Festbett. Die Reaktionslösung tritt unten aus dem Reaktor heraus und wird von dort aus der Aufarbeitung zugeleitet.

Die Mengenregelung für die Einspeisung wird üblicherweise über ein pneumatisches Stellventil (2) und einem Durchflußmesser (3) vorgenommen. Die Einspeisetemperatur liegt im Bereich von 55-62°C, die Ablauftemperatur im Bereich von 75-85°C. Der Reaktor wird adiabatisch gefahren; Wärmeverluste werden durch Isolierung und Begleitheizung vermieden. Der Druckverlust durch das Lewatitbett wird im oberen Teil des Reaktors (4) gemessen. Aus Sicherheitsgründen wird die Einspeisung des Reaktionsgemisches bei einem durch das Lewatitbett hervorgerufenen Druckverlusts von 2 bar abgeschaltet.

Die gewichtsmäßige Zusammensetzung des in den Reaktor eingespeisten Reaktionsgemisches kann in folgenden Bereichen variieren: Phenol 75-85 Gew.-%, Bisphenol-A und Nebenprodukte 12-20 Gew.-%, Aceton 2-6 Gew.-%.

### Beispiel 1 (Vergleich)

In einen Reaktor gemäß Fig. 1 mit 25 m² Querschnittsfläche und einer Höhe der zylindrischen Einheit von 5 m werden 100 m³ phenolfeuchtes, nicht modifiziertes Harz mit einem Vernetzungsgrad von 2 % eingefüllt. Der Reaktor wird mit einer Reaktionsgemischmenge von 17 m³ /h beaufschlagt (Einspeisetemperatur 60°C). Folgende Leistungsdaten wurden ermittelt:

| | |
|---|---|
| Druckverlust im Reaktor: | 1,7 bar |
| Actonumsatz im Reaktor: | 96,5 % |
| Selektivität der Reaktion: | 96 % |

Eine Erhöhung der Einspeisemenge bei dieser Reaktorschüttung ist nicht mehr möglich, da einige hundert Liter mehr einen exponentiellen Druckanstieg und damit die Auslösung der Sicherheitsabschaltung zur Folge haben.

### Beispiel 2 (Schichttyp a):

In einen Reaktor gemäß Beispiel 1 werden 80 m³ phenolfeuchter, nicht modifizierter Ionenaustauscher mit einem Vernetzungsgrad von 2 % gefüllt und anschließend mit 20 m³ eines phenolfeuchten, nicht modifizierten Harzes mit dem Vernetzungsgrad 4 % überschichtet.

Folgende Leistungsdaten der Harzschüttung bei der BPA-Synthese wurden ermittelt:
1. Einspeisemenge Reaktionsgemisch 17 m³ /h (60°C)

| | |
|---|---|
| Reaktordruckverlust: | 0,1 bar |
| Acetonumsatz: | 96,0 % |
| Selektivität der Reaktion: | 95,1 % |

2. Einspeisemenge Reaktionsgemisch 20 m³ /h (60°C)

| | |
|---|---|
| Reaktordruckverlust: | 0,15 bar |
| Acetonumsatz: | 93,0 % |
| Selektivität: | 94,5 % |

### Beispiel 3 (Schichttyp a, modifiziert):

In einem Reaktor gemäß Beispiel 1 werden 80 m³ phenolfeuchtes, nicht modifizierter Ionenaustauscher mit einem Vernetzungsgrad von 2 % gefüllt und anschließend mit 20 m³ eines phenolfeuchten Harzes mit einem Vernetzungsgrad von 4 %, dessen Sulfonsäuregruppen zu 3 Mol-% mit Mercaptoethylamin belegt worden sind überschichtet. Folgende Leistungsdaten der Harzschüttung bei der BPA-Synthese wurden ermittelt:

Einspeisemenge Reaktionsgemisch 20 m³ /h (60° C)

| | |
|---|---|
| Reaktordruckverlust: | 0,15 bar |
| Acetonumsatz: | 96,9 % |
| Selektivität der Reaktion: | 97,9 % |

### Beispiel 4 (Schichttyp b)

In einen Reaktor gemäß Beispiel 1 werden 80 m³ phenolfeuchter, nicht modifizierter Ionenaustauscher mit einem Vernetzungsgrad von 2 % gefüllt und anschließend mit 20 m³ eines phenolfeuchten, 2 % vernetzten Harzes, dessen Sulfonsäuregruppen zu 15 Mol-% mit Mercaptoethylamin belegt worden sind, überschichtet. Folgende Leistungsdaten der Harzschüttung wurden ermittelt:
1. Einspeisemenge Reaktionsgemisch 17 m³ /h (60° C)

| | |
|---|---|
| Reaktordruckverlust: | 0,25 bar |
| Acetonumsatz: | 99,9 % |
| Selektivität der Reaktion: | 98,5 % |

2. Einspeisemenge Reaktionsgemisch 23 m³ /h (60°C)

| | |
|---|---|
| Reaktordruckverlust: | 0,6 bar |
| Acetonumsatz: | 97,5 % |
| Selektivität der Reaktion: | 98,0 % |

## Patentansprüche

1. Ionenaustauscherschüttungen zur Erhöhung der Raum-Zeit-Ausbeute der Festbettreaktoren bei der Bisphenol A-Herstellung aus Phenol und Aceton in zylindrischen Festbettreaktoren, die mit sulfonsauren Ionenaustauscherharzen von gelförmigem oder makroporösem Typ gefüllt sind, dadurch gekennzeichnet, daß
a) die untere Schicht der Schüttung aus einem niedrigvernetzten Harztyp (Vernetzungsgrad (≤2 %) besteht und volumenmäßig 75-85 % der Gesamtschüttung ausmacht und
b) die obere Schicht der Schüttung mit einem Volumenanteil von 15-25 % entweder aus einem höhervernetzten Harztyp (Vernetzungsgrad ≥2 % ≤4 %), dessen Sulfonsäuregruppen zu 1-25 Mol-% mit Alkyl-SH-Einheiten enthaltenden Spezies (ionisch fixiert) belegt sein können, besteht oder
c) aus einem niedrigvernetzten Harz (Vernetzungsgrad ≤2 %), dessen Sulfonsäuregruppen zu 1-25 Mol-% mit Alkyl-SH-Einheiten enthaltenden Spezies (ionisch fixiert) belegt sind.

## Claims

1. Ion exchanger packings to increase the space/time yield of fixed bed reactors in the production of bisphenol A from phenol and acetone in cylindrical fixed bed reactors, which are filled with sulphonic acid ion exchange resins of the gel or macroporous type, characterised in that
a) the lower layer of the packing consists of a slightly crosslinked grade of resin (degree of crosslinking ≤ 2%) and constitutes 75-85% of the volume of the complete packing and
b) the upper layer of the packing, constituting 15-25% of the volume, consists either of a more highly crosslinked grade of resin (degree of crosslinking ≥ 2% ≤ 4%), 1-25 mol.% of the sulphonic acid groups of which may be modified by (ionically attached) species containing alkyl-SH units or
c) of a slightly crosslinked resin (degree of crosslinking ≤ 2%), 1-25 mol.% of the sulphonic acid groups of which are modified by (ionically attached) species containing alkyl-SH units.

## Revendications

1. Distributions d'échangeuses d'ions pour augmenter le rendement espace-temps des réacteurs à lits fixes dans la préparation de Bisphénol-A à partir de phénol et d'acétone dans des réacteurs cylindriques à lits fixes qui sont remplis avec des résines échangeuses d'ions d'acides sulfoniques du type en forme de gel ou macroporeux, caractérisées en ce que
a) la couche inférieure de la distribution est constituée par un type de résine faiblement réticulée (degré de réticulation ≤2%) et représente de 75 à 85% en volume de la distribution totale, et
b) la couche supérieure de la distribution représentant une fraction volumique de 15 à 25% est constituée, soit d'un type de résine à réticulation supérieure (degré de réticulation ≥2% ≤4%) dont les groupes d'acides sulfoniques peuvent être recouverts (fixés par voie ionique) à concurrence de 1-25 moles % avec une espèce contenant des unités alkyl-SH, soit
c) d'une résine faiblement réticulée (degré de réticulation ≤2%) dont les groupes d'acides sulfoniques sont recouverts (fixés par voie ionique) à concurrence de 1-25 moles % avec une espèce contenant des unités alkyl-SH.
